Europäisches Patentamt

European Patent Office

Office européen des brevets

(11) Publication number: **0 315 289**
**A2**

## EUROPEAN PATENT APPLICATION

(21) Application number: **88202611.5**

(22) Date of filing: **04.11.88**

(51) Int. Cl.4 **A61K 37/02**

(30) Priority: **06.11.87 US 118185**

(43) Date of publication of application:
**10.05.89 Bulletin 89/19**

(84) Designated Contracting States:
**AT BE CH DE ES FR GB GR IT LI LU NL SE**

(71) Applicant: **ONCOGEN**
**3005 First Avenue**
**Seattle, WA 98121(US)**

(72) Inventor: **Rowe, John M.**
**9091 Olympus Beach Road N.E.**
**Bainbridge Island Washington 98110(US)**
Inventor: **Shoyab, Mohammed**
**2405 Westmont Way West**
**Seattle Washington 98199(US)**

(74) Representative: **Kinzebach, Werner, Dr. et al**
**Patentanwälte Reitstötter, Kinzebach und**
**Partner Sternwartstrasse 4 Postfach 86 06 49**
**D-8000 München 86(DE)**

(54) **Cell growth inhibitory factor.**

(57) A growth factor designated BCIF and methods for its use are provided. BCIF inhibits proliferation of cells, particularly human breast cancer cells and human melanoma cells. BCIF can be used alone or in combination with other growth factors such as a transforming growth factor-beta or Oncostatin M, which factors may interact additively or synergistically with BCIF to inhibit cell proliferation. Methods for obtaining BCIF are also provided.

EP 0 315 289 A2

## CELL GROWTH INHIBITORY FACTOR

Technical Field

This invention relates to proteinaceous growth factors that inhibit cell proliferation, particularly proliferation of human breast cancer cells.

Background

The ability to control tumor cell growth without serious side effects to the host organism has long been a goal in the continuing search for improved methods for treating cancer. It has been proposed that the proliferation of neoplastic cells may in part result from aberrant expression of polypeptide growth factors leading to autocrine stimulation of the neoplastic cell population. Identification of natural cell inhibitors that can counteract growth factors therefore may be essential to understanding and control of neoplastic cell development.

The respose of a tumor to therapeutic drugs can be variable due to tumor cell heterogeneity: All cells within a tumor may not respond equally well to a given therapeutic agent. Cell growth regulatory peptides often elicit multiple biological activites, including both growth stimulatory and inhibitory activities depending upon the target cell type. In particular, various of the growth regulatory polypeptides such as the interferons, tumor necrosis factor $\alpha$, interleukins and transforming growth factors $\alpha$ and $\beta$. have been shown to inhibit proliferation of some tumor cells. These polypeptides may act independently as well as additively and synergistically to inhibit tumor cell proliferation.

Combinations of cell growth inhibitory factors which exhibit additive and synergistic effects in vitro may function in vivo to selectively inhibit the subpopulations of tumor cells within a tumor mass, or at different metastatic sites, thereby providing overall a greater reduction in the tumor burden than is currently achievable with therapeutic drugs. It is therefore of interest to develop combinations of growth regulatory molecules which can be used in combination to inhibit tumor cell growth, thereby reducing the effective dose of the individual polypeptides and minimizing undesirable side effects which may be associated with higher concentrations of the individual polypeptides.

## RELEVANT LITERATURE

The DNA sequence for BSF-2 is reported in Hirano et al.. Nature (1986) 324:73-76 and Hirano et al., Proc. Natl. Acad. Sci. USA (1987) 84:228-231. Properties of IFN-$\beta$2. particularly its ability to promote the growth and differentiation of B cells. are described in Billiau. Immunology Today (1987) 8:84-87. The sequence of $\beta$2-IFN is reported in May et al., Proc. Natl. Acad. Sci. USA (1986) 83:8957-8961. The identity between $\beta$-INF and BSF-2 is described in Sehgal et al.. Science (1987) 235:731-732.

## SUMMARY OF THE INVENTION

A cell growth inhibitory factor, designated BCIF is provided which finds use as an inhibitor of proliferation of neoplastic cells. particularly human breast cancer cells. BCIF can be used alone or in combination with other growth factors having antineoplastic activity. BCIF is characterized as being obtainable from human lung cancer cells, as having a molecular weight of about 21,000 Daltons, and inhibiting growth of neoplastic cells. Fragments of the growth factor can be used in the production of antibodies that counteract the inhibitory action or that themselves can be used to produce anti-idiotypic antibodies which exhibit growth inhibitory activity.

## BRIEF DESCRIPTION OF THE DRAWING

Figure 1 is a graph showing increasing inhibition of cell growth as a function of increasing BCIF concentration in a breast cancer cell line.

Figure 2 is a graph showing inhibition of proliferation of a human melanoma cell line with a combination of TBF-beta and BCIF as compared to either polypeptide alone.

## DESCRIPTION OF SPECIFIC EMBODIMENTS

In accordance with the present invention, compositions comprising BCIF and fragments thereof, as well as methods for the preparation and use of these compositions, are provided. The cell growth inhibitory factor BCIF is characterized as being produced by the human lung carcinoma cell line H-2981. BCIF is further characterized as having a molecular weight of about 21,000 Daltons as determined by analytical SDS-PAGE gel electrophoresis and inhibiting growth of neoplastic cells either alone or in combination at least one other growth factor which inhibits proliferation of neoplastic cells. The combination may act additively or synergistically to inhibit cell growth.

A partial amino acid sequence of BCIF is set forth below:

| | |
|---|---|
| V-P-P-G-E-D-S-K-D-V | 10 |
| A-A-P-H-R-Q-P-L-T-S | 20 |
| S-E-R-I-D-K-Q-I-R-Y | 30 |
| I-L-D-G | 34 |

The above sequence uses the standard one-letter amino acid abbreviation for each amino acid. This amino acid sequence for BCIF is identical to amino acid residues 2-35 from the amino terminus of the BSF-2 mature protein factor reported by Hirano et al., Nature (1986) 324:73-76, which is identical to the IFN-$\beta$2 sequence reported by May et al.,Proc. Natl. Acad. Sci. USA (1986) 83:8957-8961 for amino acids 30-63.

BCIF can be obtained in a variety of ways. It is available from naturally occurring sources, such as carcinoma cell lines. An example of such a cell line is H-2981, which is a human lung carcinoma cell line described in numerous publications, such as Hellstrom et al., Proc. Natl. Acad. Sci. USA (1986) 83:7059-7063. The carcinoma cells can be grown in growth media known to support their proliferation, and BCIF can be isolated from the culture medium.

BCIF can be purified so as to be substantially free of cellular components employing various purification techniques. These techniques can include solvent extraction, gel permeation chromatography, reversed phase-HPLC, electrophoresis, and the like. A particularly useful technique is affinity chromatography using an antibody specific for a region of BCIF-2 different from BSF-2 and INF-$\beta$2. Such antibodies can be prepared using synthetic immunogens (peptides) that encompass the distinctive region. Other specific techniques are described in the following examples. For active fragments showing inhibitory activity, various synthetic techniques may be employed, where the polypeptide will be synthesized on a solid support or prepared by cleavage of entire BCIF molecules or fragments thereof. A number of commercial synthesizers are available and may be used to advantage, for example, from Smith Kline Beckman; Applied Biosystems, etc.

BCIF and fragments thereof also can be prepared by employing recombinant techniques. The amino acid sequence may be used to design a probe, and a cDNA library or genomic library from stimulated H-2981 carcinoma cells can be screened for hybridizing sequences. To enhance the likelihood of identifying the correct sequence, a cDNA library from lung carcinoma cells or other related cell lines which do not produce BCIF may be used to cross-hybridize. Those sequences from the H-2981 cells which do not hybridize to the sequences from the non-BCIF producer will be concentrated as to the desired sequence. Sequences which hybridize to the probe and do not cross-hybridize with cDNA from cells which do not produce BCIF may be screened by using Xenopus oocytes to determine if they contain the BCIF gene. An assay for the expression of BCIF using restriction fragments inserted into a prokaryotic expression vector such as λgt-11 and then screening with antibodies for BCIF to detect a cross-reactive peptide fragment, can be used.

Once the fragment containing the sequence encoding BCIF has been identified, the fragment may be manipulated by resection, primer repair, in vitro mutagenesis, restriction endonuclease digestion, or the like, to provide a 5′ transcriptional and, as appropriate, translational initiation region (if necessary or desired) and optionally a different 3' transcriptional and, as appropriate, translational termination region. Various expression vectors into which the open reading frame encoding BCIF may be inserted are commercially available or described in the literature. The vector can be transformed into an appropriate host for expression. Prokaryotic expression hosts include E. coli and B. subtilis; eukaryotic hosts include S. cerevisiae, CHO cells, monkey kidney cells, streptomyces, etc.

The subject compositions may be modified by truncating at the N- or C-terminus where up to about 10 amino acids may be removed. Biological activity can be tested as described later and active molecules retained. In addition, up to about 5 mole percent of non-conservative mutations may be made and up to 10 mole percent of conservative mutations may be made. For conservative substitutions, the amino acids may be broken down into non-polar aliphatic amino acids (G,A,P,V,I,L), polar aliphatic amino acids (C,S,T,M,N,Q), aromatic amino acids (F,H,W,Y), basic amino acids (K,R), and acidic amino acids (D,F). Conservative mutations occur within these groups and non-conservative mutations across group boundaries.

The BCIF or fragments thereof having inhibitory activity may be used as an anti-neoplastic composition. BCIF can be obtained having a relative specific activity of about 0.1 units/ng, usually at least about 20 units/ng, preferably at least about 100 units/ng, and up to complete purity. A unit is the amount of activity required to reduce the rate of cell growth and division of a target cell line (e.g., ZR-70-30) by one-half. Additionally, the antineoplastic composition may include at least one other growth factor which has antineoplastic properties, the combination of growth factors being present in a amount sufficient to inhibit proliferation of neoplastic cells. The other growth factor may be one which may act additively with BCIF to inhibit cell growth, for example Oncostatin M, or it may be one which acts synergistically with BCIF, for example, TGF-beta. The combination of growth factors has the advantage of decreasing the amount of any one peptide which must be used to obtain growth inhibition.

For use in vivo, the subject peptide, either alone or in combination with other growth factors as described above may be administered in a variety of ways, such as injection, infusion, topical or parenteral application, or the like. Administration may be in any physiologically acceptable carrier, such as sterilized water, phosphate buffered saline, saline, aqueous ethanol, etc. Administration will be designed to provide a concentration at the cellular level of 1 unit/ml, preferably at least 10 units/ml, and more preferably at least 100 units/ml of GIA. Design of administration techniques can follow the guidelines established for other biologically active proteins, such as insulin or somatostatin.

Besides being used for their growth inhibiting properties, BCIF and fragments thereof may be used in diagnostics. Thus, BCIF or fragments thereof may be joined to a label, such as a radioisotope, enzyme, fluorescer, chemiluminescer, enzyme fragment, particle, etc. A wide variety of diagnostic assays are known, see, for example, U.S Patent Nos. 3,791,932; 3,817,837; 4,134,792; 3,996,345. The subject compounds also may be used for the production of antibodies, either polyclonal or monoclonal, where the antibodies may be used for detection of BCIF in diagnostic assays, for studying the effects of BCIF on cells in culture, for detecting the presence of BCIF in conditioned media, and the like. Again, guidelines established for other proteins, such as insulin, provide guidance in the production of antibodies against BCIF and its fragments and the selection and use of such antibodies in assays. In addition, antibodies against BCIF can be used in the production of anti-idiotypic antibodies that can mimic the activities of the original BCIF molecule. Individual fragments of the BCIF molecule find use in the preparation of a mixture containing fragments of 10-20 peptides in length and overlapping so as to cover the full length of the BCIF molecule. Such mixtures can be used in the production of antibodies against the peptide fragments, particularly monoclonal antibodies, and the thus-produced antibodies can be screened against BCIF itself to select antibodies having the proper binding affinities. The technique can also be used with other proteinaceous molecules having biological activity.

The invention now being generally described, the same will be better understood by reference to the following examples which are set forth for purposes of illustration and are not to be considered limiting of the invention unless so specified.

EXPERIMENTAL

EXAMPLE 1

Biological Activity Assays

1. Growth Inhibition Assay

ZR-70-30 human breast carcinoma cells (ATCC CRL1504; American Type Culture Collection, Rockville, Maryland) were used as the sensitive indicator cell line. The assays were performed in 96-well flat-bottomed tissue culture plates (Costor 3596). Approximately $3.5 \times 10^3$ cells in 0.05 ml of IMDM/DMEM (50:50 v/v) supplemented with 7.5% fetal bovine serum (FBS) and penicillin streptomycin (P'S) (growth medium) were added to each well with the exception of the outer wells, to which was added 0.1 ml of sterile PBS. Three hours later, 0.05 ml of the test samples dissolved in growth medium was added to each well. Plates were incubated at 37°C for 3 days in a humidified 5% $CO_2$ - 95% air atmosphere for 3 days. Cells were then pulsed for 18 hrs with 0.05 ml of 5-[$^{125}$I] iodo-2'-deoxyuridine (IdU) (0.05 μCi/well; Amersham IM.355V). One unit of growth inhibitory activity (GIA) is defined as the amount of the factor required to inhibit $^{125}$[I] IdU incorporation by 50%.

2. Synergistic Growth Inhibition Assay

A375 melanoma cells (ATCC CRL1619) American Type Culture Collection, Rockville, Maryland) were used as the sensitive indicator cell line. The assays were performed in 96-well flat-bottomed tissue culture plates (Costor 3596). Approximately $3.5 \times 10^3$ cells in 0.05 mL of DMEM supplemented with 10% fetal bovine serum (FBS) and penicillin/streptomycin (P'S) (growth medium) were added to each well with the exception of the outer wells, to which was added 0.1 mL of sterile PBS. Three hours later, 0.025 mL of the test samples dissolved in growth medium was added to each well. Plates were incubated at 37°C for three days in a humidified 5% $CO_2$ - 95% air atmosphere for three days. Cells were then pulsed for 18 hrs with 0.05 mL of 5-[$^{125}$I] iodo-2'-deoxyuridine (IdU) (0.05 μCi/well; Amersham IM.355V), to determine the degree of inhibition of cell proliferation.

EXAMPLE 2

Isolation and Purification of BCIF from a Human Lung Carcinoma Cell Line, H-2981

(a) Isolation

H-2981 cells were cultured and grown to confluency in 20 roller bottles (850 cm²) (Corning C2540) in IMDM supplemented with 15% Fetal Bovine Serum (FBS) and penicillin streptomycin (P S). When the cells reached confluency, they were washed once with serum-free DMEM (100 ml) and incubated for 15 days in serum-free DMEM (50 ml) with the medium being collected and replenished every 3 days. The conditioned medium was centrifuged to remove cellular debris and frozen at -20°C until 5 liters of medium had been collected. The conditioned medium was concentrated using Amicon filtration, molecular weight cutoff of 10,000. The retentate (600 ml) was dialyzed (molecular weight cutoff 3,500) at 4°C for 2 days against 4 liters of 10 mM Tris, pH 8.0 with three changes of dialysis buffer. The retentate was centrifuged (30 min at 30,000 g), and the supernatant was collected and referred to as crude conditioned medium.

(b) Purification

(i) Anion Exchange Chromatography

The crude conditioned medium containing approximately 600 mg of protein was loaded onto a column of DEAE-Sephacel (5x2.5 cm) (Pharmacia) at a flow rate of 3 ml/min. The column was then washed with 40 column volumes of 10 mM Tris, pH 8.0 (primary buffer). and equilibrated in 10 mM Tris buffer, pH 8.0. Absorbed proteins were eluted with a linear gradient of 0-0.5 M NaCl in 10 mM Tris buffer, pH 8.0. Five ml fractions were collected. Absorbance at 280 nm was determined, and aliquots of fractions were tested for growth inhibitory activity (GIA) as described. Those fractions which contained GIA (fractions 24-28) were pooled. The majority of the GIA eluted from the DEAE column as a single peak, at a NaCl concentration of 0.15 M.

(ii) Reversed-phase High Performance Liquid Chromatography C-18A

Pooled DEAE-Sephacel fractions containing GIA were adjusted to 0.2% TFA and further purified using a μ-Bondapack C-18 column. Approximately 31.25 mg of the DEAE pool were applied isocratically to a C-18 column (7.8x300 mm), equilibrated in 0.1% TFA, at room temperature, at a flow rate of 1.0 ml/min and chart speed 0.1 cm/min. Elution conditions were achieved with linear gradients of 0-45%, 45-52% and 52-100% of acetonitrile in 0.1% TFA: 0-45% in 10 min, flow rate 1 ml/min; 45-52% in 420 min, flow rate 0.2 ml/min; and 52-100% in 20 min, flow rate 1 ml/min. Fractions (1 ml) were collected between 45-52% acetonitrile and assayed for GIA. The majority of GIA eluted at 48% acetonitrile.

(iii) C-18B

Pooled fractions (45-49) from the C-18(A) step above containing the majority of GIA were diluted 3-fold with 0.1% TFA and were rechromatographed using the same μ-Bondapack C-18 column as above. This step further separated the BCIF from the bulk of UV absorbing material.

Approximately 0.18 mg of protein were loaded onto the 1. C-18 column equilibrated in 0.1% TFA. The pooled fractions were loaded onto the column at room temperature at a flow rate of 1 ml/min. The chart speed was 0.1 cm/min. Elution conditions were achieved with linear gradients of 0-25% and 25-35% n-propanol in 0.1% TFA. The gradient conditions were 0-25% in 10 min, flow rate 0.8 ml/min; and 25-35% in 300 min, flow rate 0.4 ml/min. Fractions (1 ml) were collected between 25-35% n-propanol and assayed for GIA.

(iv) Gel Permeation Chromatography

The final step in the purification of the BCIF was achieved using two Bio sil TSK-250 columns (300x7.5 mm) arranged in tandem. Pooled fractions (51-54) from the C-18B step were concentrated to 0.1 ml using a speed VAC concentrator (Savant) to which had been added an equal volume of acetonitrile containing 0.1% TFA. Approximately 0.015 mg of C-18(B)-purified protein was applied to the tandem columns which were then eluted with 50% acetonitrile containing 0.1% TFA. The flow rate was 0.4 ml/min and chart speed was 0.25 cm/min. Absorbance was monitored at 214 nm. Fractions (0.4 ml) were collected and assayed for GIA. The GIA eluted as a single peak with proteins of $M_r$ 26,000. Protein markers were ovalbumin ($M_r$ 45,000), chymotrypsinogen ($M_r$ 25,000), ribonuclease ($M_r$ 13,700) and insulin ($M_r$ 6,000). The purification of BCIF was approximately 5,800-fold with an overall recovery of 7.4% (see Table I). The half-maximal dose of purified BCIF was 9 pg protein.

(V) Polyacrylamide Gel Electrophoresis

Polyacrylamide slab gels in the presence of NaDodSO₄ were prepared as described (Laemmli, Nature - (1970) 227:680-685). The stacking gel (4%) and resolving gel (15%) were prepared from stock solutions of acrylamide (30% v/v) and N,N'-methylenebisacrylamide (0.8 w/v). Gels were cast using a Bio-Rad vertical mini-slab gel unit. The proteins were visualized by silver staining. A single band of $M_r$ 21,000 was observed.

TABLE I

| Summary of Purification of BCIF | | | | |
|---|---|---|---|---|
| Purif. Step | Protein (mg) | GIA Units x $10^{-6}$ | Specific Activity Units x $10^{-3}$ 'mg | Yield % |
| Crude | 600.000 | 12.50 | 0.0002 | 100.0 |
| DEAE | 31.250 | 21.00 | 0.0067 | 168.0 |
| C-18(A) | 0.180 | 3.22 | 0.1790 | 24.0 |
| C-18(B) | 0:015 | 1.27 | 0.8500 | 10.1 |
| TSK-250 | 0.008 | 0.93 | 1.1600 | 7.4 |

EXAMPLE 3

Characterization of BCIF

(a) Amino-terminal Amino Acid Sequence of Purified BCIF

Two preparations of BCIF (25 and 30 pmol, respectively) were purified and subjected to automated Edman degradation, using a Model 470A protein sequencer (Applied Biosystems, Inc.). The amino-terminal amino acid sequence of BCIF is as follows;

V-P-P-G-E-D-S-K-D-V        10

A-A-P-H-R-Q-P-L-T-S        20

S-E-R-I-D-K-Q-I-R-Y        30

I-L-D-G                          34

(b) Dose-response of Purified Growth Inhibitory Activity

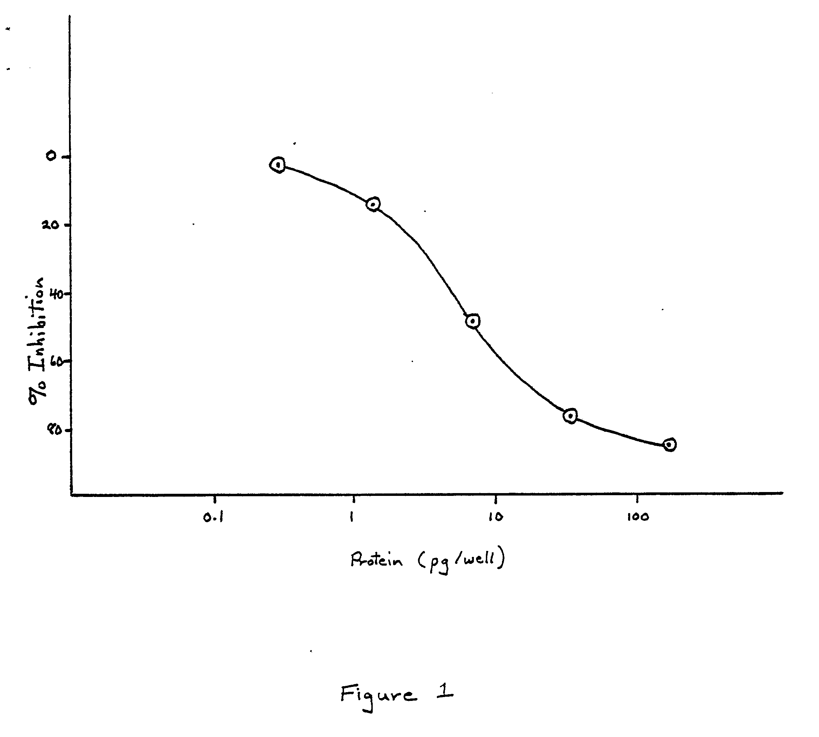

A purified preparation of BCIF obtained from the Bio-sil TSK-250 column was assayed at multiple dilutions using the human mammary carcinoma cell line, ZR-75-30, as the indicator cell. Inhibitory activity is shown by a decrease in cell growth and division (increase in % inhibition) when plotted versus increasing BCIF concentration as shown in Figure 1.

(c) Synergistic and Additive Effects of BCIF and Other Growth Factors

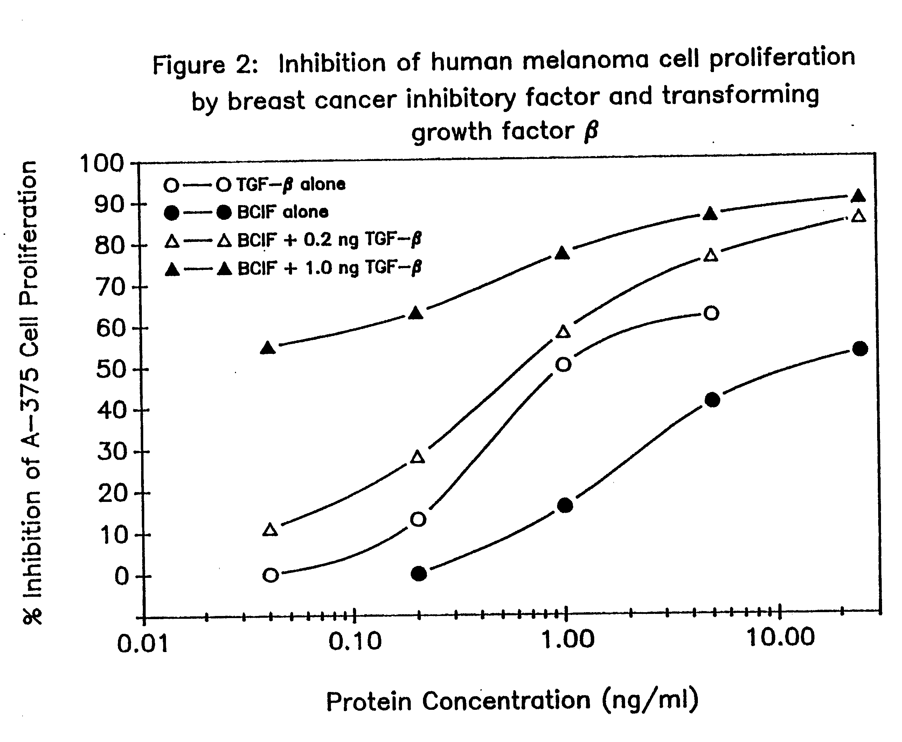

The growth inhibitory activity of highly purified BCIF, TGF-$\beta$, and Oncostatin M were determined and possible synergy between BCIF and TGF-$\beta$ and BCIF and Oncostatin M in inhibiting the proliferation of A375 melanoma cells. As shown in Figure 2, BCIF and TGF-$\beta$ added individually to the culture dishes, inhibited A-375 cell proliferation over the range of 0.2-25 ng/ml and 0.04-5 ng/ml, respectively. In the presence of low amounts of TGF-$\beta$ (0.2 ng/ml), the potency of BCIF was increased by about 10-fold and the maximal inhibition of BCIF was raised from 55% to 85% (Fig. 2). A synergistic effect was obtained with the combination of BCIF and TGF-$\beta$. The degree of enhancement was approximately two-fold the expected additive response. For example, cell proliferation was inhibited 16% by 1 ng/mL BCIF and 13% by 0.2 ng/mL TGF-$\beta$, which in combination inhibited 58%, two-fold the expected additive effect of 29%. Furthermore, in the presence of BCIF alone (1.0 ng/ml) the observed inhibition of 16% was raised to 27% in the presence of a low concentration of TGF-$\beta$ (0.04 ng/ml) which by itself had no measurable effect.

In contrast, BCIF (1 ng/mL) and Oncostatin M (1 ng/mL) inhibited A375 cell proliferation by 16% and 32% respectively, and in combination by 46%, which approximates the predicted additive effect.

The compositions, and methods for their use, of the subject invention provide a means for inhibiting growth of neoplastic cells. BCIF, either alone or in combination with other growth factors having antineoplastic activity, inhibits proliferation of tumor cells in vitro and may be useful in vivo, particularly against tumors comprising cells having differential sensitivity to conventional antineoplastic agents.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

The invention now being fully described, it will be apparent to one of ordinary skill in the art that many changes and modifications can be made thereto without departing from the spirit or scope of the appended claims.

## Claims

1. The use of a composition comprising at least one polypeptide characterized as capable of inhibiting proliferation of neoplastic cells, wherein one of said polypeptides is a first polypeptide having an amino acid sequence substantially similar to at least eight consecutive amino acids included in the following sequence:

V-P-P-G-E-D-S-K-D-V        10

A-A-P-H-R-Q-P-L-T-S        20

S-E-R-I-D-K-Q-I-R-Y        30

I-L-D-G        34

for preparing a pharmaceutical composition for inhibiting proliferation of neoplastic cells.

2. The use of Claim 1, wherein said first polypeptide is human BCIF.

3. The use of claims 1 or 2, wherein said first polypeptide comprises from about 8 to 20 amino acids and is capable of inhibiting the proliferation of ZR-75-30 carcinoma cells or A-375 cells.

4. The use of anyone of claims 1 to 3, wherein said composition further comprises a second polypeptide.

5. The use of Claim 4, wherein said second polypeptide acts synergistically with said first polypeptide to inhibit proliferation of said neoplastic cells.

6. The use of Claim 5, wherein said second polypeptide is a transforming growth factor-beta.

7. The use of Claim 4, wherein said second polypeptide acts additively with said first polypeptide to inhibit proliferation of said neoplastic cells.

8. The use of Claim 7, wherein said second polypeptide is Oncostatin M.

9. A composition useful for inhibiting proliferation of neoplastic cells, comprising at least one polypeptide characterized as having antineoplastic activity, wherein one of said polypeptides is a first polypeptide comprising BCIF.

10. The composition according to Claim 9, wherein said composition further comprises a second polypeptide.

11. The composition according to Claim 10, wherein said second polypeptide comprises a transforming growth factor-beta or Oncostain M.

12. The cell growth inhibitory factor BCIF and fragments thereof.

Figure 1

Figure 2: Inhibition of human melanoma cell proliferation by breast cancer inhibitory factor and transforming growth factor $\beta$